# EUROPEAN PATENT SPECIFICATION

(11) **EP 1 452 155 B1**
(45) Date of publication and mention of the grant of the patent: **26.03.2008**
(21) Application number: 04251054.5
(22) Date of filing: 26.02.2004
(51) Int. Cl.: A61F 5/02, A61G 7/10

(54) **Back support pad**
Rückenstützunterlage
Coussin de support de dos

(30) Priority: 27.02.2003 CN 03106669
(43) Date of publication of application: 01.09.2004
(73) Proprietor: Wong, Cho Kee, Homantin, Kowloon, Hong Kong (CN)
(72) Inventor: Wong, Cho Kee, Homantin, Kowloon Hongkong (CN)
(74) Representative: Powell, Timothy John

(56) References cited:
- EP-A- 0 827 730
- US-A- 4 926 845
- US-A- 4 991 573
- US-A- 5 122 111
- US-A- 5 450 858
- US-A- 5 547 461
- US-A- 5 560 046
- US-A- 5 651 763

## Description

### Background of the Invention

This invention relates to an orthopaedic device of the type used to correct postural habits of the wearer to reduce the risk of lower back pain and injury, as well as to support the back when the wearer is carrying a heavy load such as a soldier's or hiker's back pack or a student's book bag. More particularly, this invention, which is designed to be both a preventive and therapeutic device, relates to an orthopaedic device to influence the postural habits of the user in the form of a belt adapted to be worn by a user, with or without back packs, or loops or pouches for carrying tools or hiker's or soldier's supplies.

Many orthopaedic devices have been proposed in the past and the stated purpose of them was to provide lower back support or relieve lower back pain, typically to reduce the risk of heavy lifting. All prior inventions related to interaction with the mechanics of the spine and supporting structure, but none related to actually changing the posture of the wearer. Belts designed for this purpose have generally been from three different types-elastic girdle-type corsets, nylon belts which encapsulate some foam material, and leather belts, which are more commonly seen in weightlifting. All of these are intended to support the muscular structure, while conforming to the existing posture of the wearer. They may or may not support one or more of the mechanics of posture, but they do not necessarily change the wearer's posture while doing so.

US Patent number 5,651,763 discloses a lumbosacral support system comprising a belt and an orthopaedic pad carried by the belt. The pad has a contoured template surface with a transversely extending central trough portion for accommodating the protruding spinal process of the wearer when the pad is pressed against the lumbar regions, and a pair of raised plateau regions flanking the central trough portion for contacting the erector spinae muscles of the wearer to provide support. The template surface of the pad has a vertical surface contour through the central region approximating the average lordotic curve of a wearer. The belt is longitudinally tapered so the pad is arranged at an angle to vertical to optionally engage the lumbar region of the wearer.

There is much documentary evidence finding poor posture, rather than just lifting, is the primary cause of most back injuries. Poor posture, as discussed in "repetitive stress injury" (RSI) research, gradually weakens the spinal structure, rendering it vulnerable to failure in the future. Other back support styles promote their ability to support underlying tissues; e.g. muscles and ligaments and/or create intra-abdominal pressure, which minimises compressive forces to the spine. No other device can represent that the lumbar spine of the wearer will adapt to remain in its most efficient posture.

The lumbar spine is a concave structure, which is most efficient when in this contour. A normal spinal curve in the lower back is known as lumbar lordosis, and is known to present the strongest structure. Lumbar lordosis makes it possible for the erector spinae muscles, which are attached to the spine, to stabilise the vertebral column during flexion, acting in contrast to abdominal muscles and the action of gravity. If the spinal column was straight, the erector spinae muscles in conjunction with the abdominal muscles would only be able to apply opposing forces perpendicular to the spine, causing the spine to have to support the entire weight of the body, which would quickly wear out and damage the spine. In an analogy to an archery bow, the compression provided by the string maintains the curve of the bow. The string must be cut to elongate the bow. So too, with a subject having an abnormal lumbar lordosis, such as a flatter curve, the erector spinae muscles must elongate to accommodate the flatter curve, and consequently, their range-of-motion restriction mechanism is compromised. Thus, the supporting musculature is most efficient when the spine it supports is in lordosis. A spinal column with a flatter lumbar curve is believed to be the primary cause of injury and pain.

Injury and pain may occur to the muscles in the back in the form of over strained muscles, ruptured blood vessels supplying the muscles, torn ligaments, etc. Injury to the spinal column, resulting from putting stress on the spinal column when the spine is not curved in its natural slight S-shape, can be manifested as a slipped or ruptured disc, in which the soft nucleus pulposus in the core is squeezed out and presses on a nerve, causing a pinched nerve, or it restricts the movement of the vertebrae required for lateral bending or normal degree of rotation of the spine. A wrong working posture in standing or sitting, especially if one bends forward too much, will strain the erector spinae and will also produce an uneven pressure on the discs, which are the resilient cushions between the vertebrae in the spine. As noted above, when the discs are "squeezed" out of shape, the surrounding tissue will compress or stretch. If the pressure becomes excessive, one may end up with a slipped or ruptured disc.

By the same token, if one bends backward too much, which increases curvature of the spine beyond natural lumbar lordosis, a condition known as hyperextension may result in which the spinal discs are squeezed to a degree which causes the protruding surface of the disc to push against a nerve, causing pain. This is especially true with pregnant women who bend backwards to compensate for their centre of gravity from being pulled forward by the baby's weight. Likewise, the more overweight a person is the greater is the tendency to lean backward to compensate for their centre of gravity being pulled forward. Furthermore, some people with spinal pain or injury in the cervical lordosis (neck region of the spine) region of the spine have a tendency to lean backwards to lessen the pressure on the neck vertebrae and discs in order to reduce the pain. Whether the person bends backward due to pregnancy or to reduce neck spinal pain or simply as a result of bad posture, this condition usually does not cause lower back pain while one is bending backwards. Since there is no increased pain to cause one to correct this poor posture, the abuse to the lower back may continue until lower back pain or injury to the lower spine with severe pain occurs. Thus, this new orthopaedic device, which has many beneficial functions can correct un-conscientious poor posture, and prevent pain and injury to the spine. It should be noted that a critical review of Musculoskeletal Disorders and Workplace Factors by the U.S. Department of Health and Human Services (DHHS), National Institute for Occupational Safety and Health (NIOSH), which was published by the Centres for Disease Control (CDC) in DHHS (NIOSH) Publication No. 97-141, concluded that "there is strong evidence that working groups with high levels of static contraction, prolonged static loads, or extreme working postures involving the neck/shoulder muscles are at increased risk for neck/shoulder MSDs [musculoskeletal disorders]" It is to be expected that good lower back posture, which is maintained by this back support belt will also contribute to better posture in the shoulder and neck area of the spine.

Stress on the spine as a result of poor posture during one's daily work or sports activities manifests itself as lower back pain during the day, and especially at the end of the day. If this improper stressing of the spine continues, the repetitive stress injury gradually weakens the spinal structure, rendering it vulnerable to failure in the future. A case-referent study was conducted in an automotive assembly plant to evaluate the health effect of trunk postures, such as bending and twisting, that deviate from anatomically neutral. The journal article describing the study titled "Back disorders and non-neutral trunk postures of automobile assembly workers," published in Scand J. Work Environ 1991; 17: 337-346, found that "back disorders were associated with mild trunk flexion, severe trunk flexion, and trunk twist or lateral bend. The risk increased with exposure to multiple postures and duration of exposure."

Posture in most people is a subconscious habit, done without thinking. Other than extensive training, improving posture must be done at the subconscious level. Nerves and muscles will react to a firm stimulus, such as accupressure or massage.

### Object of the Invention

It is the object of the present invention to overcome or substantially ameliorate at least one of the above disadvantages and/or more generally to provide an improved back support belt incorporating a back support pad.

### Disclosure of the Invention

The invention provides a back support belt in accordance with claim 1 of the appended claims.

There is disclosed herein a back support belt comprising an orthopaedic pad for positioning against the lumbar spine formed substantially of resilient, yet substantially non-compressive posture-supportive material, having a contoured body-facing surface adapted to conform to and/or maintain normal lumbar lordosis of the lower spine, and having a vertical groove at the centre thereof the vertical groove being adapted to acommodate the wearer's lower spine in use, a strap extending from opposite ends of the orthopaedic pad for extension therefrom about the torso of the wearer in use, and four magnetic strips, two of which being arranged on either side of the vertical groove in the centre of the orthopaedic pad and running parallel to the groove, the other two being also arranged on either side of the vertical groove and being perpendicular to the groove, all the magnetic strips having alternating polarities so as to assist the wearer's blood vessels to cross through fields created between opposite magnetic polarities, thereby enabling the magnetic field to attract and repel charged particles in the blood to create movement and heat.

Preferably the orthopaedic pad is convex in transverse cross-section.

Preferably the orthopaedic pad further comprises a relatively high-density foam or injection moulded plastic core covered by a covering material.

Preferably the covering material comprises a plurality of breathing apertures enabling air to pass into and out of the foam core.

Preferably the back support belt further comprises loops and pouches for tools and supplies.

Preferably the strap is substantially non-elastic.

Preferably the strap further comprises one or more elastic edge inserts.

Preferably the edge inserts are provided in pairs at laterally opposed positions of the strap.

Preferably the edge inserts are substantially triangular.

Preferably the strap is supplemented by a secondary strap of greater or lesser elasticity.

Preferably the strap is in two parts interconnected by mechanical fasteners.

In this invention, the lumbar pad is firm, to resist conforming to the spinal posture of the wearer, yet shaped in a normal lordotic arc to provide a fixed reference for the spine to conform. When the firm orthopaedic pad, which is shaped to fit the natural curve of the spine, is held in contact with the lower back region by a belt worn around the waist, the pad provides a resistive but yieldable support surface. Furthermore, flexion is opposed by the natural tendency of the proprioceptors in the muscles and ligaments of the back to conform to the smooth surface. Yet another function of this orthopaedic appliance is to protect the back from improper loading caused by sudden quick movements such as occurs when one spills hot coffee on one's lap. In such situations, one reacts so quickly that the protective pain reflex system does not have time to prevent one from making the sudden motion causing improper loading on the spine. This is another example of how the back support belt maintains good posture during both unconscious and conscious activities of the wearer.

An important aspect of this invention is that the belt with the firm, lordotically shaped orthopaedic pad, is worn lower on the waist than belts that have here-to-fore been available. As such good posture is maintained as the user changes back and forth from standing to sitting positions. The other types of back support belts are typically worn higher up on the waist and cover a greater area of the upper torso and restrict motion of too much of the torso structure. Although the belts which have here-to-fore been available may support one or more of the mechanics of good posture while the user is standing, they are so rigid that the user generally rotates out of good posture when sitting down. Thus this new belt is also well suited for pregnant women, especially from four months to full term, in which the baby is carried in the upper abdomen. As noted above pregnant women bend backwards to compensate for their centre of gravity from being pulled forward by the baby's weight, which can cause hyperextension of the lower spine. The new belt is easily worn diagonally under the belly, helping to carry the excess weight because of its non-stretch design, and may help reverse the tendency of pregnant women from leaning backwards, and improperly stressing the spine.

Another feature which improves the fit and comfort of the back support belt is the addition of elastic wedges on each side of the firm belt. Although the size and location of these elastic wedges in the back support belt may vary, in the preferred embodiment, the elastic wedges are 1.0 to 5.0 cm wide at the base and 1.5 to 4.0 cm in vertical length, and the apexes of the wedges are directly opposed to each other, with the elastic wedges being located 2.0 to 6.0 cm from the lateral edges of the orthopaedic pad. These selectively placed elastic wedges allow for some resistive expansion of the belt for more comfortable fitting and breathing of the wearer, especially when the wearer changes from standing to sitting positions, without compromising the belt's function of keeping the lordotically shaped orthopaedic pad firmly in contact with lumbar spine to provide a resistive but yieldable support surface. Thus, to avoid the pitfalls with conventional back support belts in which large elastic panels are placed on the sides of the belt or when the entire belt is elastic, or when an elastic pad or other type of soft conforming pad contacts the spinal curve, the high modulus elastic side wedges will not defeat the ability of the firm but lordotically shaped orthopaedic pad in this new belt to provide the proper support surface.

Yet another feature that is incorporated into the back support belt is the addition of magnets into the orthopaedic pad. Magnets are becoming more medically accepted form of therapy for pain and healing and are commonly used on sports injuries and chronic pain conditions such as tendonitis, sprains, wrist pain, chronic lower back pain, overuse syndrome and elbow pain. Magnets, have been shown to have a therapeutic effect in reducing pain and in promoting the healing of strained muscles by promoting blood circulation and heating of body sites, and may be placed in the orthopaedic pad in strips containing magnets. It is believed that when blood vessels cross through a magnetic field created between opposite magnetic polarities (north and south magnetic poles) that the magnetic field attracts and repels charged particles in the blood creating movement and heat, thereby contributing to the healing process. The reduction in pain may be attributed to more rapid healing, and possibly to a blockage of pain caused by stimulation of the central nervous system by the small electrical current that may be induced in the body site. Magnets may be produced by embedding ferrite particles into rubbery or plastic sheets with concentrations of ferrite particles of different polarities to form geometric rows or columns of magnetic strips with alternating polarities. Furthermore, thin ferrite magnetic bars or circular magnets or magnets of other geometric shapes may be produced and placed into flexible strips of rubber or plastic materials or into fabrics. In all cases, it is desirable to have alternating opposite magnetic polarities between successive magnetic bars, circular rings or with other geometric configurations of magnets. Fabric strips containing magnets may be a made of woven, knitted or non-woven fabrics, with two or more fabric layers sewn together or adhered by an adhesive, with the magnets encapsulated between the inner and outer layers of the strip. Alternatively, the strips containing the magnets may be made of plastic or rubber of a foamed or injection moulded material with the magnets embedded in the material of the strip. Thin strips containing magnets may be bonded to the inside surface of the orthopaedic pad or to the belt fabric covering the orthopaedic pad so that the magnet strips are encapsulated between the fabric covering the outside surface of the orthopaedic pad and the lordotically curved inner surface of the orthopaedic pad.

The lordotically shaped orthopaedic pad may be produced from any material which is firm but yieldable to the back muscles, such as closed cell foams, plastics or other materials. The orthopaedic pad worn by non-invalid individuals, should have a hardness of 35-40°, with a preferred hardness of 38°. For invalid patients, a broader range of hardness values ranging from 20-40 would be utilised to provide a firm but softer surface for the more fragile patients, with the preferred hardness range being 25-35. The orthopaedic pad may be compression moulded, but the preferred method of manufacturing the pad is by injection moulding, which allows for more precise shape and higher production rate. The pad may be ventilated with small holes for greater wear comfort. Furthermore, the groove is formed in the centre vertical part of the pad to accommodate the protruding part of the spin.

Since the orthopaedic belt is comfortable to wear around the waist and prompts the wearer to move the lumbar forward to the proper curvature and keeps the lumbar in the proper position even when conscious or subconscious sudden motions and lateral inputs to the L4 to L5 regions of the spine are experienced by the wearer, this new belt is ideal for invalid patients. Not only is this back support belt beneficial to the patient while in a sling, it will also be helpful in properly restricting the lying and sitting posture of the patient to the proper position with out stressing the back, particularly if the patient may have a seizure. The back support belt may be worn while lying in bed, and would be especially beneficial when transporting the patient from the bed to a chair or from the bed or chair to and from a sling. Furthermore, if the patient regains some motor skills required for walking, the new back support belt would be most useful in helping to support the back and maintain good posture to prevent injury to.the back while the patient is healing and regaining strength. A study to support the benefits to the patient is discussed in DHHS (NIOSH) Publication No. 97-141 notes that for occupational injuries involving days away from work, the U. S. Bureau of Labor Statistics reported in 1994 that "approximately 705, 800 cases (32% [of reported cases]) were the result of overexertion or repetitive motion." This NIOSH publication also reported that there was "strong evidence that low-back disorders are associated with work-related lifting and forceful movements...and strong evidence of an association between exposure to WBV [whole body vibration] and low-back disorder." Furthermore, the NIOSH review "provided evidence that work-related awkward postures are associated with low-back disorders."

### Brief Description of the Drawings

A preferred form of the present invention will now be described by way of example with references to the accompanying drawings, wherein
Figures 1A to 1D are 3-dimensional illustrations of an orthopaedic pad showing a perspective view and views from the front, top and side respectively,
Figures 2A to 2C are perspective views of the orthopaedic pad when rotated and also showing the shape of the planar surface opposite the lordotic shaped surface;
Figures 3A to 3B are perspective views of the orthopaedic belt, with magnets, ventilation holes, elastic wedges in the belt for better fitting, mechanical belt fasteners for the .primary belt and a secondary belt for additional support;
Figure 4 is a more detailed illustration showing the location of magnet strips and ventilation holes in the pad, elastic wedges in the belt, and how the belt fabric is constructed to cover the orthopaedic pad;
Figure 5 is a front view of the orthopaedic pad showing the lordotic shape of the pad, the groove to accommodate the protruding part of the spine, and the fabric encapsulating the pad;
Figures 6A and 6D consist of side view of the orthopaedic belt showing how the orthopaedic pad fits the lordotic curve of the spine, whereas Figures 6B and 6C are included as a basis of comparison;
Figures 7A to 7C show how tools and pouches can be attached to the back support belt in which a leather belt with a belt buckle may be used for the belt portion of the orthopaedic device for better support in moving and lifting;
Figures 8A to 8D show how the belt helps support a hunter's load and improves his posture, as well as how the hunter's back pack is attached to the orthopaedic support belt;
Figures 9A to 9D show how the belt helps a student carrying a heavy book pack with better posture and how the book pack is attached to the back support belt;
Figures 10A to 10C show how the back support belt improves the posture of a pregnant woman, as well as an over weight person;
Figures 11A to 11C shows how the orthopaedic back support belt supports a person lifting weights, in which the belt portion of the orthopaedic device is made of leather with a belt buckle for greater support;
Figures 12A to 12C show a soldier wearing the orthopaedic back support device with a leather belt and belt buckle for greater support and with clip on pouches for personal gear and supplies;
Figures 13A to 13C shows how the orthopaedic belt can be worn to improve posture in carrying a heavy bag or pack, which may also be worn with the soldier's gear shown in Figures 12A to 12C;
Figure 14 is a depiction of a patient sling which may be used with a back support belt of the present invention lifting an invalid;
Figure 15 is a depiction of a patient sling with closed back support belt;
Figure 16 is an illustration of a patient sling with fabric loop for holding and securing back support belt; and
Figure 17 is an illustration of a patient sling with the orthopaedic back support belts of the invention inserted.

### Description of the Preferred Embodiments

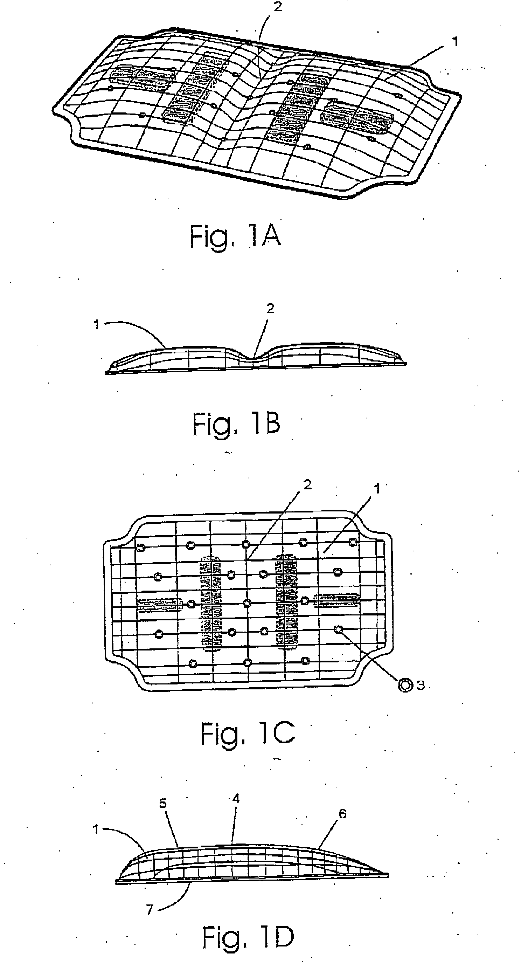

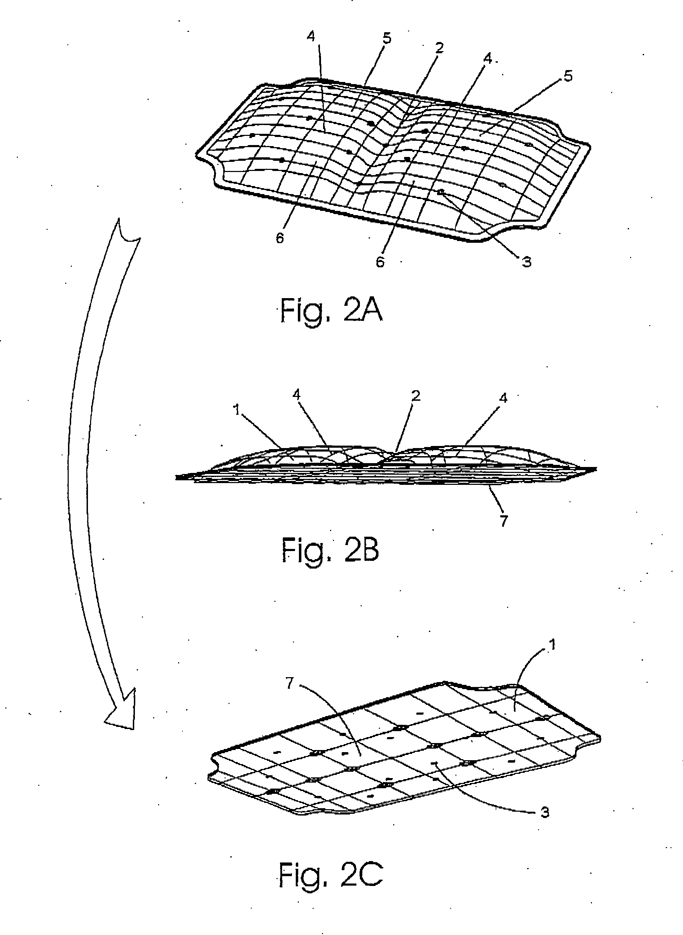

Referring now to the Figures 1 to 13 of the drawings in which like reference characters designate like or corresponding parts throughout the several views. Figure 1a shows a 3-dimensional perspective of the orthopaedic pad, providing a view of the template 1 of the orthopaedic pad and of the groove 2 in the centre of the orthopaedic pad to accommodate the protruding part of the spine. Figure 1b depicts a front view of the orthopaedic pad in which the template 1 of the pad and the groove 2 to accommodate the spine are further illustrated. Figure 1c provides a top view of the template 1 of orthopaedic pad showing the location of the groove 2 for the spinal protrusion, as well as ventilation holes 3 for providing ventilation for the orthopaedic pad. Multiple air holes; which maybe circular in diameter, or in any shape, traverse through the thickness of the pad to allow air to enter and to allow moisture to evaporate from the wearer, and thereby improve the thermal comfort of the orthopaedic pad. Other features of the orthopaedic pad which can be seen in Figure 1a, Figure 1b and Figure 1c, are described in the remaining Figures. Figure 1d provides a side view of the orthopaedic pad revealing its lordotic shape 4 to fit the normal lumbar lordosis of the lower spine. The lordotic shape of the pad fitting the upper lumbar fitting surface 5 and the lower lumbar fitting surface 6 are clearly shown. Also in Figure 1d, the flat planar surface 7 of the orthopaedic pad opposite to the lordotic surface 4 worn against the back of the wearer is shown. In Figure 2a, Figure 2b and Figure 2c provide another perspective of the orthopaedic pad when it is rotated. Possible locations of the air holes can be seen in Figure 2a and Figure 2c. The groove 2 accommodating the protruding part of the spine can be seen in Figure 2a and Figure 2b. The lordotic shape 4 of the orthopaedic pad showing the surface fitting the upper lumbar 5 and the surface fitting the lower lumbar 6 is depicted in Figure 2a.

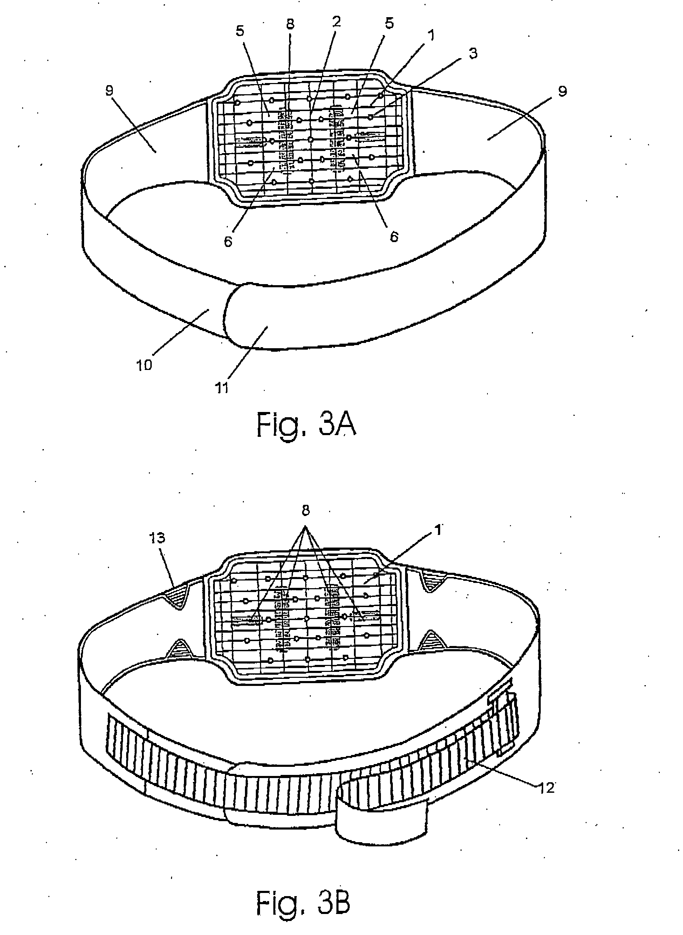

Figure 3a is a perspective view showing the orthopaedic belt, depicting the.template 1 of the orthopaedic pad, magnets 8, ventilation holes 3, and a waist belt 9 attached to and supporting the orthopaedic pad in a number of different ways. The waist belt may be made of any material including polyester, nylon, polypropylene, polyethylene or natural or synthetic leather, and the construction of the belt may be woven, knitted or non-woven. However, materials and fabrication methods are preferred which form a strong, dimensionally stable belt which fits tightly but comfortably around the waist when fastened, and which holds the lordotic shaped orthopaedic pad firmly in contact with the lower back of the wearer.

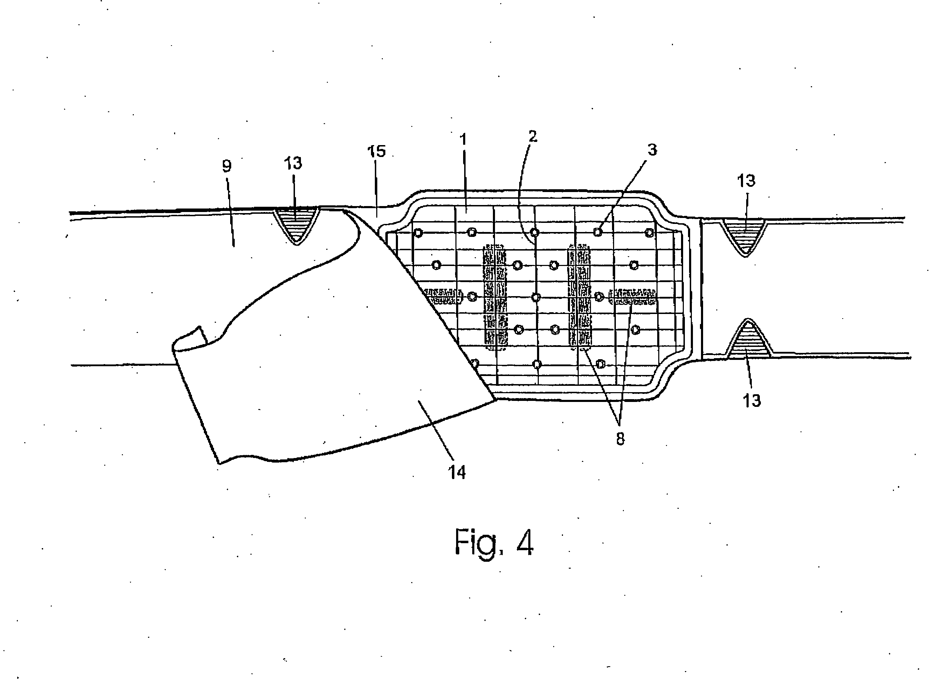

One type of woven construction that may be used for the belt fabric is "rip stop" in which nylon or polyester yarns are woven to produce a diagonal over-weave designed to prevent rips or tears from spreading. The fabric may be folded or layered .with other types of belting fabrics and sewn together near the edges of the belt to construct a belt of the desired thickness and widths along the length of the belt from the orthopaedic pad to the belt ends 10 and 11 as shown in Figure 3a and Figure 3b. In Figure 4, the.inside and outside layers of the belt widen in the area of the orthopaedic back pad to encapsulate the back pad, which may be wider than the waist belt. The fabrics covering both sides of the orthopaedic pad may also be different fabrics from the fabric of the waist-line belting, which may then be sewn to the waist-line belting, or the fabric encapsulating the orthopaedic pad may be attached to the waist-line belt by adhesives or by mechanical means such as zippers or buttons. The latter mechanical fastening technique affords the wearer the flexibility of removing a pad that has been heavily soiled with dust or contaminants or with perspiration, as well as the opportunity to interchange different types of preventive and therapeutic orthopaedic pads. The belting fabrics shown in Figure 3a, Figure 3b and Figure 4 may also be constructed with a braided structure such as that used in braided rugs or shoe laces.

The two ends of the belt in Figure 3a, 10 and 11, may be attached by mechanical fasteners such as the "hook and loop" type used in Velcro®, which may be sewn or adhered onto the ends of the belt with an adhesive. One of the belt ends, 10 or 11, would have the "hook" structure affixed to it and the other end would have the "loop" structure affixed to it. Alternatively, the belt ends could be fastened by buttons or by a belt buckle. In Figure 3b, the waist-line belt 9 is fabricated to have a secondary belt closure 12 to be tightened and fastened over the fastened belt ends 10 and 11, to reinforce the belt closure on the wearer to prevent the belt from slipping or becoming unattached during more strenuous activities by the wearer. The secondary belt closure 12 may be permanently affixed to the ends of the waist-line belt, or it may be attached to the ends of the waist belt 9 by such means as buttons or zippers so that it may be removed from the waist-line belt when not needed.

As shown in Figure 3b, Figure 4 and Figure 6a, optional elastic wedges 13 may be placed in the waist belt 9 to improve the fit and comfort of the back support belt. Although the size and location of these elastic wedges 13 in the back support belt may vary, in the preferred embodiment, the elastic wedges are 1.0 to 5.0 cm wide at the base and 1.5 to 4.0 cm in vertical length, and the apexes of the wedges are directly opposed to each other, with the elastic wedges being located 2.0 to 6.0 cm from the lateral edges of the orthopaedic pad 1. These selectively placed elastic wedges allow for some resistive expansion of the belt for more comfortable fitting and breathing of the wearer, especially when the wearer changes from standing to sitting positions, without compromising the belt's function of keeping the lordotically shaped orthopaedic pad firmly in contact with lumbar spine to provide a resistive but yieldable support surface. Thus, to avoid the pitfalls with conventional back support belts in which large elastic panels are placed on the sides of the belt or when the entire belt is elastic, or when an elastic pad or other type of soft conforming pad contacts the spinal curve, the high modulus elastic side wedges will not defeat the ability of the firm but lordotically shaped orthopaedic pad in this new belt to provide the proper support surface.

Magnets, have been shown to have a therapeutic effect in reducing pain and in promoting the healing of strained muscles by promoting blood circulation and heating of body sites, and are placed in the orthopaedic pad in strips containing magnets 8 as is more clearly shown in Figure 3a, Figure 3b and Figure 4. It is believed that when blood vessels cross through a magnetic field created between opposite magnetic polarities (north and south magnetic poles) that the magnetic field attracts and repels charged particles in the blood creating movement and heat, thereby contributing to the healing process. The reduction in pain may be attributed to more rapid healing, and possibly to a blockage of pain caused by stimulation of the central nervous system by the small electrical current that may be induced in the body site. Magnets may be produced by embedding ferrite particles into rubbery or plastic sheets with concentrations of ferrite particles of different polarities to form geometric rows or columns of magnetic strips with alternating polarities. Furthermore, thin ferrite magnetic bars or circular magnets or magnets of other geometric shapes may be produced and placed into flexible strips of rubber or plastic materials or into fabrics. In all cases, it is desirable to have alternating opposite magnetic polarities between successive magnetic bars, circular rings or with other geometric configurations of magnets. Fabric strips containing magnets may be a made of woven, knitted or non-woven fabrics, with two or more fabric layers sewn together or adhered by an adhesive, with the magnets encapsulated between the inner and outer layers of the strip. Alternatively, the strips containing the magnets may be made of plastic or rubber of a foamed or injection moulded material with the magnets embedded in the material of the strip. Thin strips containing magnets may be bonded to the inside surface of the orthopaedic pad or to the belt fabric covering the orthopaedic pad so that the magnet strips are encapsulated between the fabric covering the inside of the orthopaedic pad and the lordotically curved inner surface of the orthopaedic pad. However in two preferred embodiments, the strips containing magnets are embedded in the inside surface of the orthopaedic pad to be flush with the lordodically curved inner surface of the firm lumbar support pad. In the second preferred embodiment, the strips containing magnets may be embedded into the outside surface 4 in Figure 1d, Figure 2a and Figure 2b of the orthopaedic pad or adhered to the outside flat surface 7 in Figure 1d, Figure 2b and Figure 2c of the lumbar support pad, or attached to the outer layer of fabric covering the back flat surface 7. As shown in Figure 1a, Figure 1c, Figure 3a, Figure 3b and Figure 4, the strips containing magnets in any of the geometries described above are arranged on the lumbar support pad with a strip on each side of the vertical groove 2 in the centre of the orthopaedic pad, and located at a distance of 1 to 7 cm from the centre line of the groove and a strip containing magnets in any of the geometries described above is also placed perpendicular to the magnetic strips running parallel to the groove in the centre of the lumbar support pad and generally would be centred between the vertical magnetic strip on each side of the groove and the edge of the orthopaedic pad joining the waist-line belt 9. This will better assure that blood vessels in the lower back cross magnetic fields of opposite polarity to achieve the maximum therapeutic effect of the magnets on the lower back. Alternatively, the ferrite particles or solid magnets of the different geometries described above may be imbedded directly into the lumbar support pad or adhered to either side of the orthopaedic pad facing the lower back or they may be adhered to be back of the orthopaedic pad.

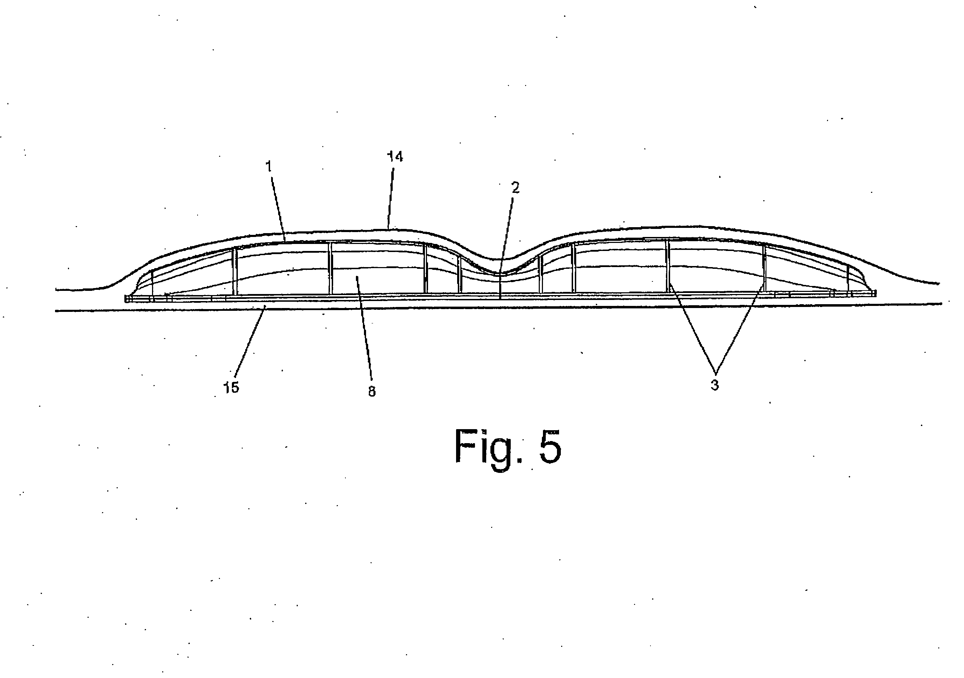

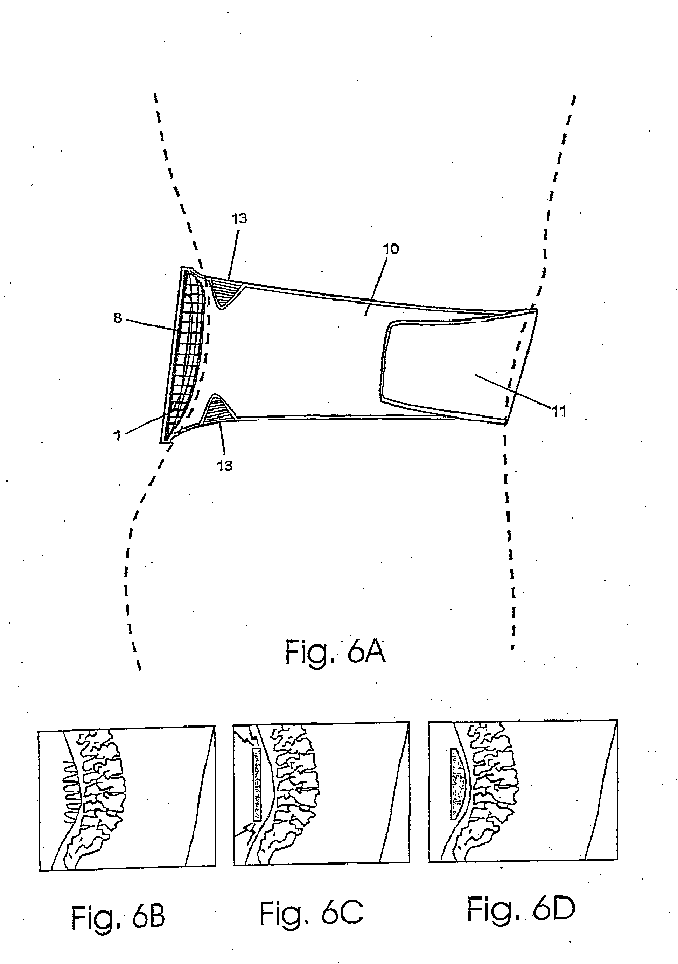

In Figure 5, a more detailed drawing of the front view of the lumbar support pad and covering is shown. The front view of the orthopaedic pad template 1, groove 2 to accommodate the protruding part of the spine, the fabric covering over the lordotic side 14 of the orthopaedic pad and fabric covering over the flat outer plane 15 are shown. As depicted in Figure 5, the orthopaedic pad may be encapsulated between the covers 14 and 15. Figure 6a is a side view of the multi-functional orthopaedic belt showing how the orthopaedic pad 1 fits the lordotic curve of the spine. The ends of the waist belt 10 and 11 are depicted and the shape and approximate location of the optional elastic wedges 13 are shown. As shown in Figure 6b, conventional elastic and soft orthopaedic pads, which are intended to support the back, conform to the existing posture of the wearer. As shown in Figure 6c stiff leather belts do not conform to the shape of the lumbar. On the other hand if the leather belt is flexible, the same situation as depicted in Figure 6b would result. Only the lordotically shaped orthopaedic pad of this invention conforms as depicted in Figure 6d to the normal lordotic curve of the lower spine, which allows the orthopaedic pad to be held in contact with the lower back region by a belt worn around the waist, which provides a resistive but yieldable support surface.

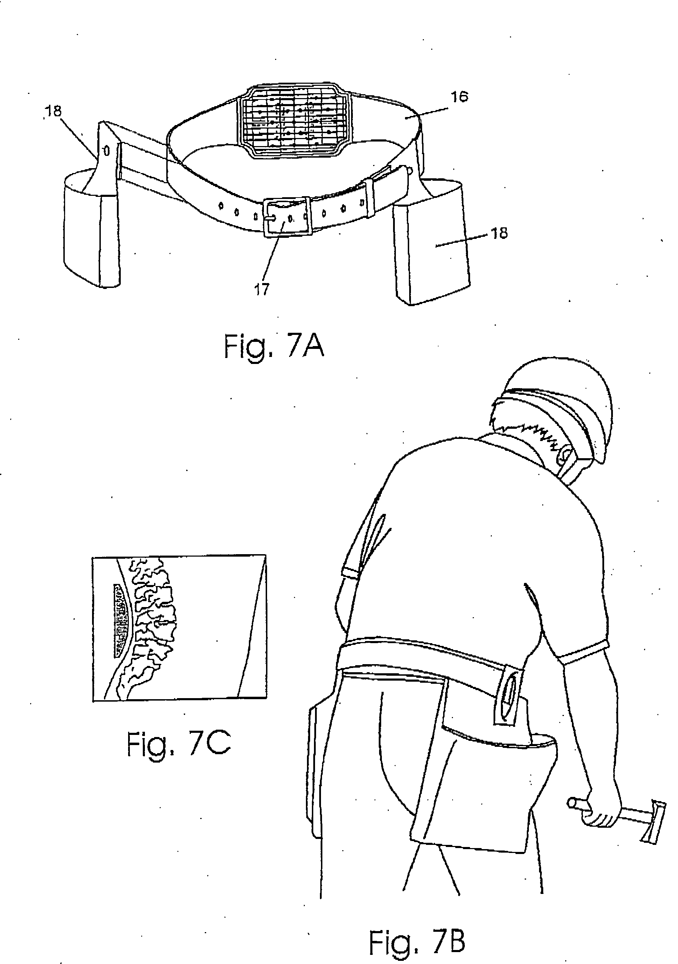

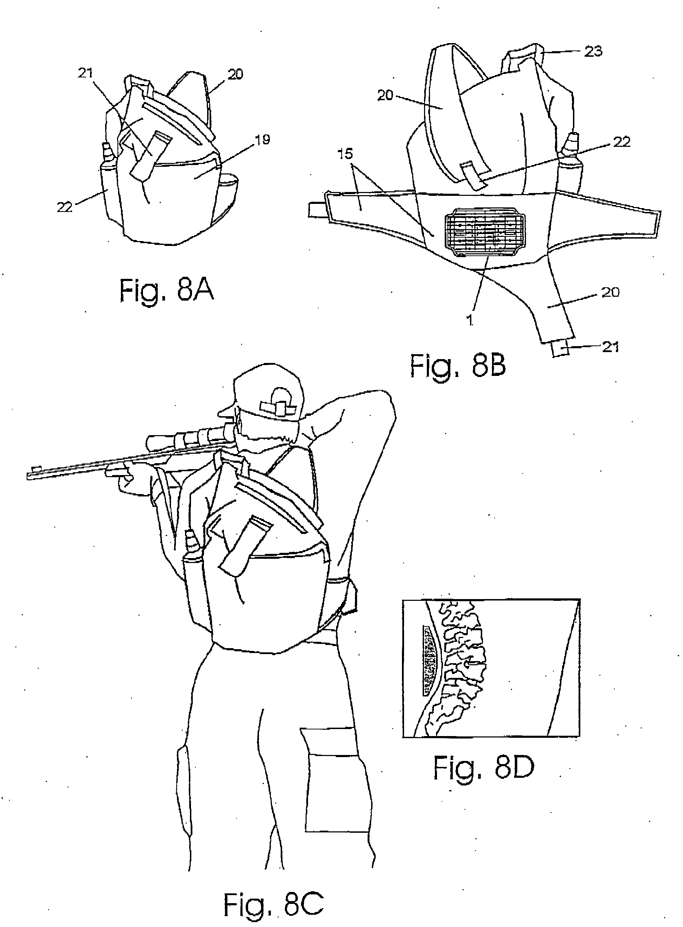

Figure 7 shows how tools and pouches 18 may be attached to the back support belt in which a leather belt 16 with a belt buckle 17 may be used for the belt portion of the orthopaedic device for better support in moving and lifting. A hunter's backpack 19 is shown in Figure 8a with body strap 20 and mechanical fasteners 21 for closure of pouches. Figure 8b shows that the orthopaedic pad 1 is attached to the back-pack. The back layer of fabric 15 (shown by dotted lines) encapsulating the orthopaedic pad 1 may actually be the part of the backpack fabric worn against the hunter's back with the fabric 14 covering the lordotic curved surface of the orthopaedic pad being sewn onto the back pack fabric as well. The belt 9, which may be made of leather may have fasteners such as those described above for Figure 3a and 3b, or may have a belt buckle 17 as described above for Figure 7. Alternatively, the back pack may be constructed as described above for Figure 3a and 3b or for Figure 7a, and attached to the backpack by buttons, zippers or other means that will allow the back pack to be removed from the backpack or reattached to the backpack as desired. Figure 8c shows how the new belt helps support a hunter's load and improves his posture, as well as how the hunter's back pack is attached to the orthopaedic support belt.

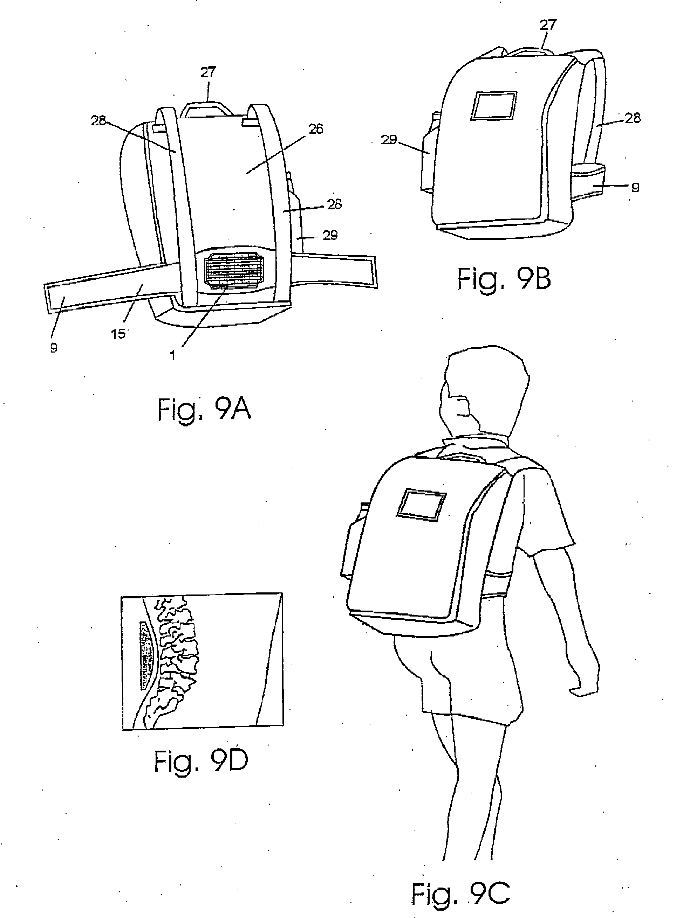

Likewise, Figure 9a, Figure 9b and Figure 9c show similar components for carrying supplies, water and cellular phone on a school child's book backpack, and how the book backpack, which may be integrated with the back support belt as described above for Figure 9, is worn by the school child in a manner that both helps support load and ensure good posture to prevent back injury.

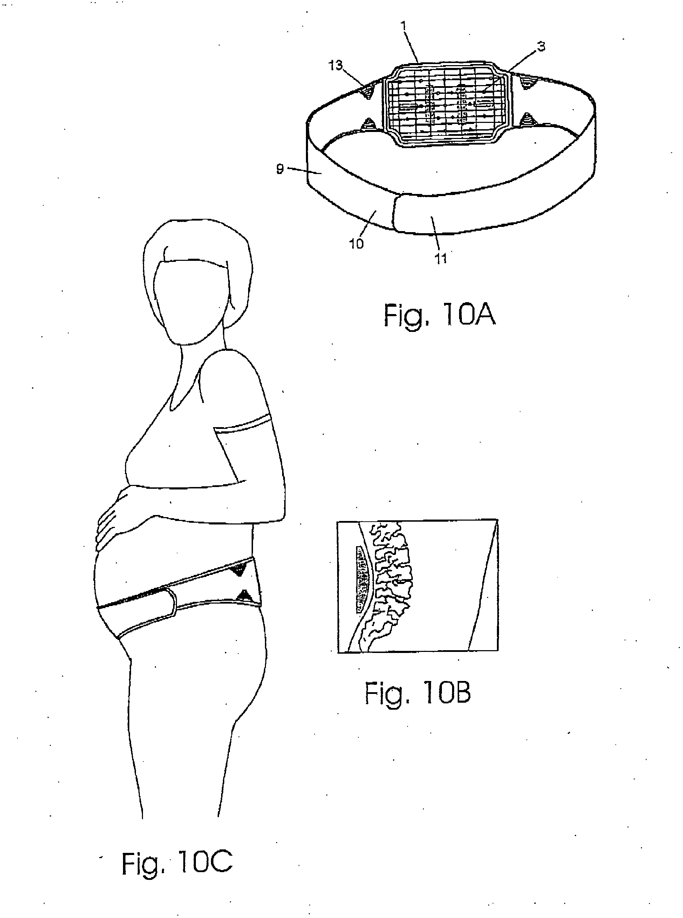

Figure 10a shows the construction of a back support belt worn by a pregnant woman or by an overweight person, which is similar in construction as the back support belt described above for Figure 3a, but the orthopaedic support belt may also be designed as depicted in Figure 3b to also have a secondary belt closure. Figure 10b shows how the new back support belt improves the posture of a pregnant woman, as well as an over weight person. Thus this new belt is also well suited for pregnant women, especially from four months to full term, in which the baby is carried in the upper abdomen. As noted above pregnant women bend backwards to compensate for their centre of gravity from being pulled forward by the baby's weight, which can cause hyperextension of the lower spine. The new belt is easily worn diagonally under the belly, helping to carry the excess weight because of its non-stretch design, and may help reverse the tendency of pregnant women from leaning backwards, and improperly stressing the spine.

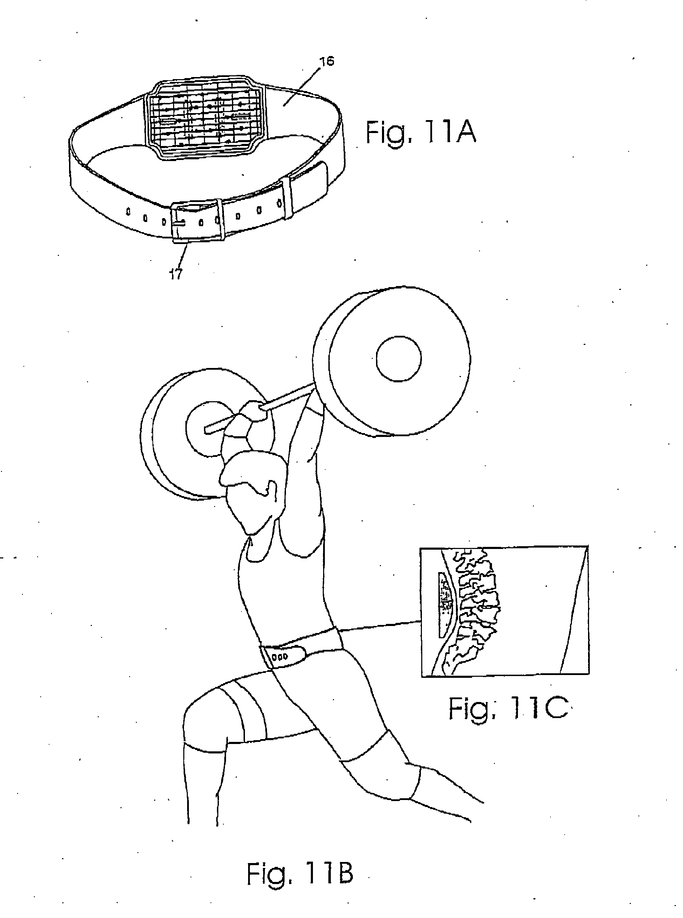

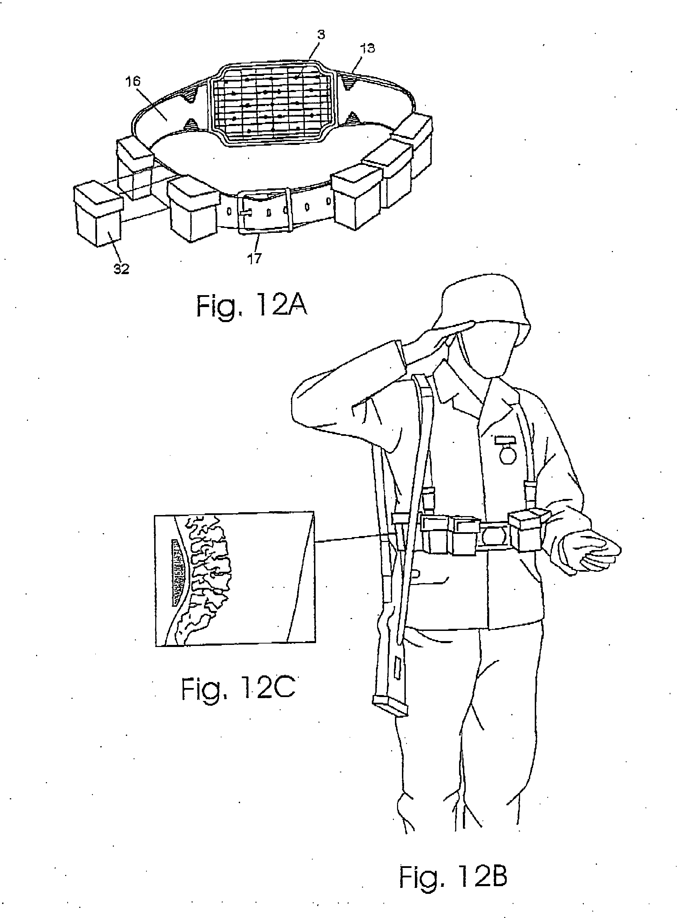

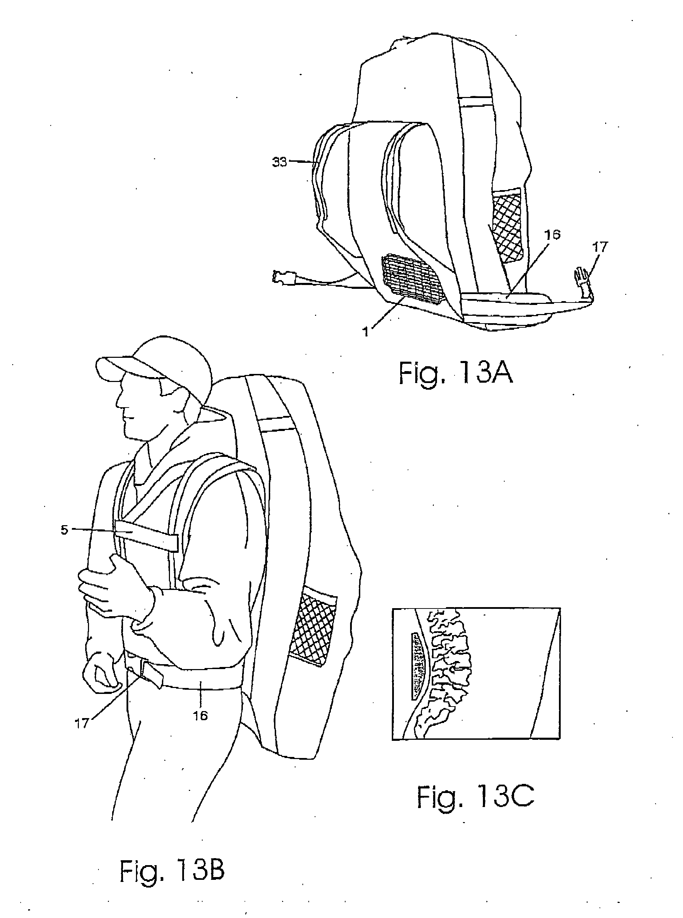

Figure 11a and 11b show how the orthopaedic back support belt supports a person lifting weights, in which the belt portion of the orthopaedic device is made of leather with a belt buckle for greater support. Figure 12 shows a soldier wearing the orthopaedic back support device with a leather belt and belt buckle for greater support and with clip on pouches for personal gear and supplies. Figure 13a and 13b show how the orthopaedic belt may be worn to improve posture in carrying a heavy bag or pack, which may also be worn with the soldier's gear shown in Figure 12. As noted above magnets are used in conjunction with the orthopaedic pad in all of these embodiments, which further enhances the functions of this orthopaedic device as both a preventive and therapeutic device.

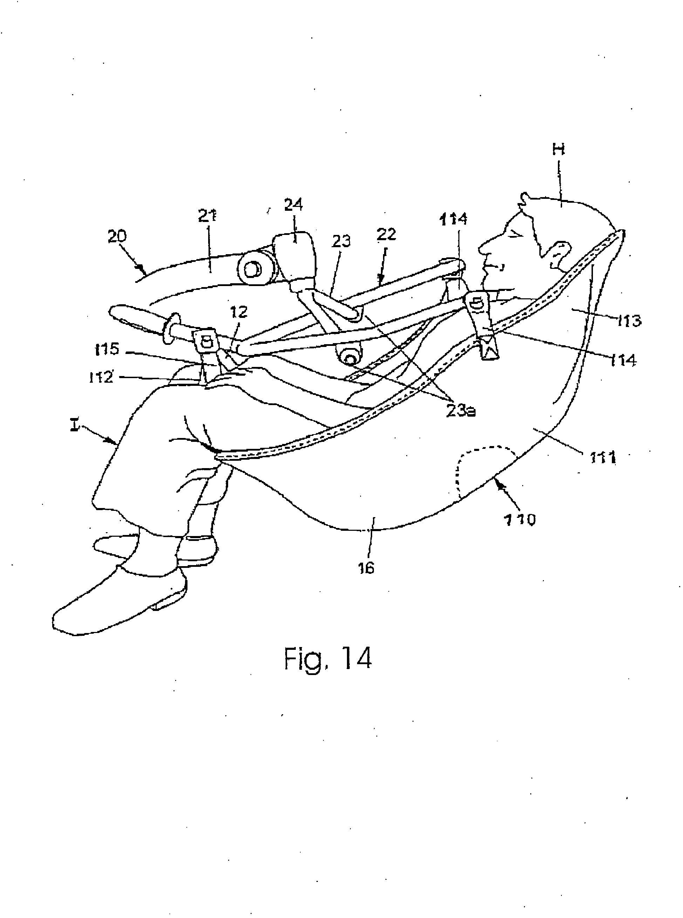

In Figure 14, there is shown a one-piece sling 110 comprising a main back portion 111 with lower end dependent leg support portions 112 and an upper end head support extension 13. The main portion 111 supports the back and shoulders of a suspended invalid I with the portions 112 respectively extending beneath and up between the thighs of the invalid whose head H is supported by the extension 113. Short extension tapes 114 providing suspension means are stitched to the main portion 111 in the shoulder regions thereof and suspension tapes 115 are similarly stitched to the ends of the leg support portions 112.

The patient sling may be made of woven, knitted or nonwoven fabrics and may also be at least partially constructed of more rigid extruded or injection moulded plastics. Further, the fabric and plastic components of the sling may be made of biodegradable or water-soluble materials. Generally patient lifting slings made of woven or knitted textiles would be considered to be durable for use multiple times by a patient or by multiple patients, until worn out by fair wear and tear, or until contaminated beyond the point of being decontaminated by laundering or other cleaning or sterilisation procedures. Typical polymeric fibres used to make woven or knitted patient slings include but are not limited to poly (ethylene terephthalate) polyester, polyamide (typically nylon 6,6), and cotton. Also, woven or knitted slings may be made of biodegradable fibres such as polylactic acid (PLA) and Eastman Chemical Company's Eastar Bio GP Copolyester, as well as other biodegradable fibres. Woven or knitted slings may also be made of fibres that are engineered to dissolve only in hot water so that the patient's urine or other warm or room temperature liquids will not dissolve the sling and make it unsafe to support the load of the patient.

However the latter woven or knitted sling could not be laundered and reused.

Although this patient lifting sling may be made of woven or knitted materials, the use of nonwoven fabrics in the lifting sling provides several additional advantages. First, it has been found that such slings can be made at a fraction of the cost of slings of woven material and will withstand the forces applied to them. It is, therefore, possible to dedicate slings to individual persons and to dispose of the slings after limited use so as to avoid the risk of cross-infection. The slings can be appropriately marked, such as with indelible ink, to ensure they are not used for other persons. Furthermore, the nonwoven materials may be made of biodegradable fibres such as polylactic acid (PLA) and Eastman Chemical Company's Eastar Bio GP Copolyester, as well as other biodegradable fibres. Nonwoven slings may also be made of fibres that are engineered to dissolve only in hot water so that the patient's urine or other warm or room temperature liquids will not dissolve the sling and make it unsafe to support the load of the patient.

The nonwoven slings may be formed by various different techniques including chemical bonding, heat bonding or hydroentangling and are typically formed of polypropylene and or polyester, neither of which, of course, are biodegradable, and would be properly disposed after one or more uses by the same patient. The nonwoven fabric can be thermal calendered or otherwise embossed to provide more texture to give the appearance of woven material.

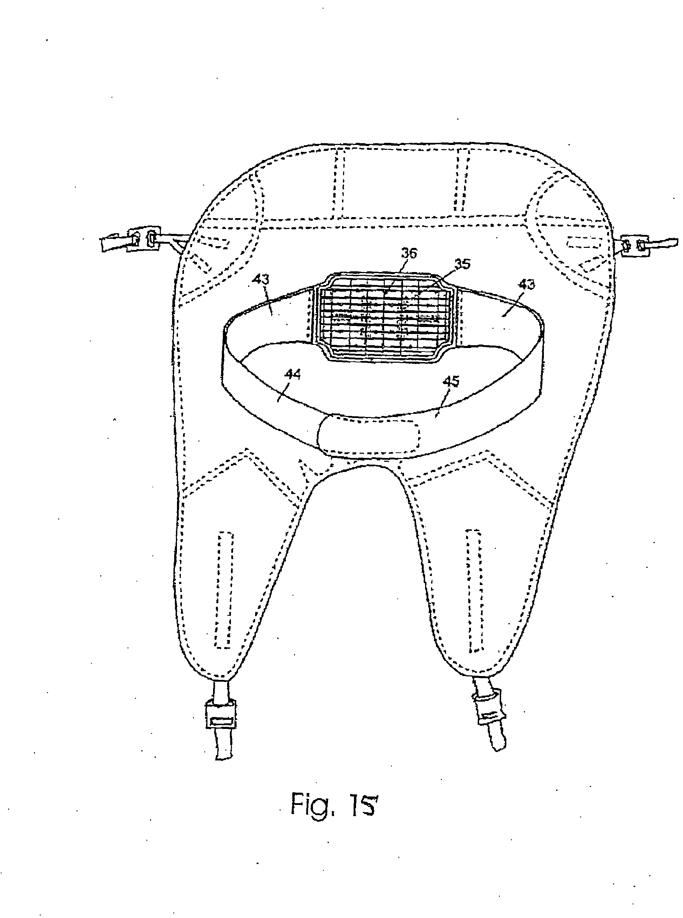

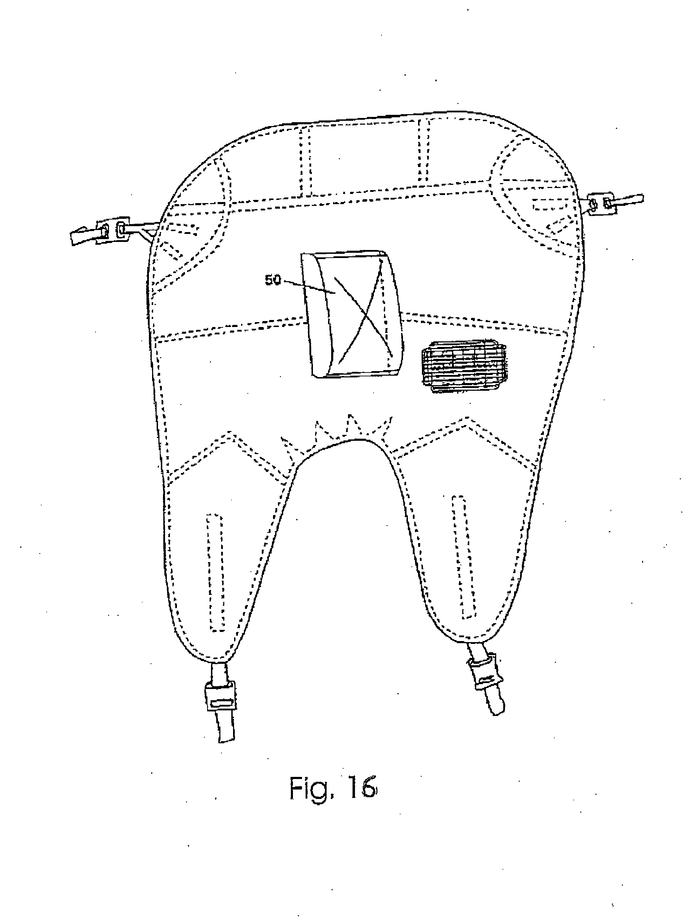

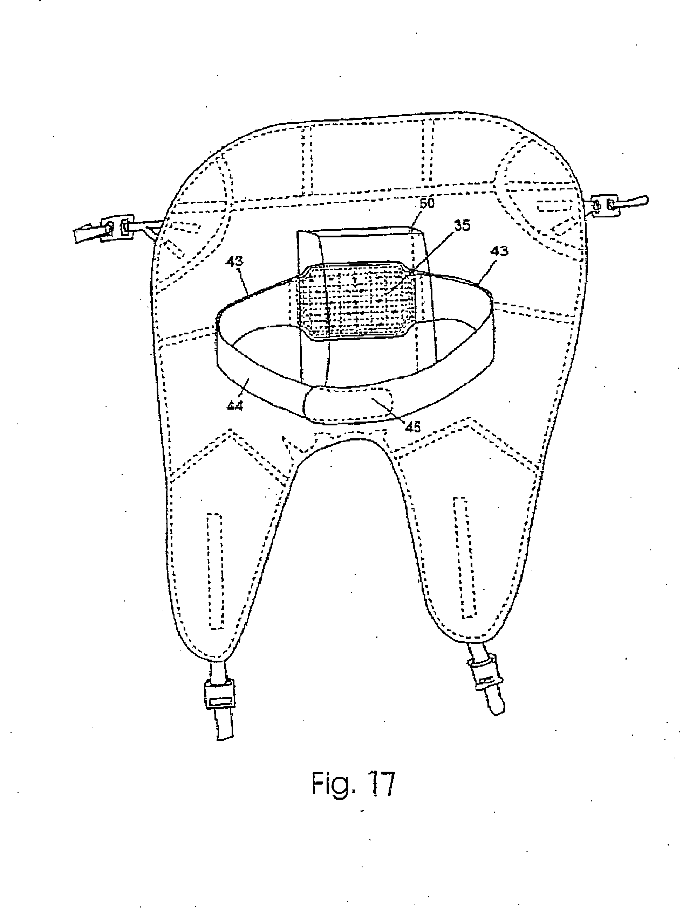

Figures 15-17 show the sling 110 incorporating the back support belt in accordance with the present invention.

## Claims

1. A back support belt comprising:
an orthopaedic pad (1) for positioning against the lumbar spine formed substantially of resilient, yet substantially non-compressive posture-supportive material, having a contoured body-facing surface adapted to conform to and/or maintain normal lumbar lordosis of the lower spine, and having a vertical groove (2) at a centre thereof, the vertical groove being adapted to accommodate the wearer's lower spine in use; and
a strap (9) extending from opposite ends of the orthopaedic pad (1) for extension therefrom about the torso of the wearer in use;
**characterised in that** the belt comprises:
four magnetic strips (8), two of which being arranged on either side of the vertical groove (2) in the centre of the orthopaedic pad and running parallel to the groove (2), the other two being also arranged on either side of the vertical groove (2) and being perpendicular to the respective magnetic strips running parallel to the groove (2), all the magnetic strips having alternating polarities so as to assist the wearer's blood vessels to cross through fields created between opposite magnetic polarities, thereby enabling the magnetic field to attract and repel charged particles in the blood to create movement and heat.

2. The back support belt of claim 1 further comprising loops and pouches (18) for tools and supplies.

3. The back support belt of claim 1 or claim 2 wherein the strap (9) is substantially non-elastic.

4. The back support belt of any one of the preceding claims wherein the strap (9) further comprises one or more elastic edge inserts (13).

5. The back support belt of claim 4 wherein the edge inserts (13) are provided in pairs at laterally opposed positions of the strap (9).

6. The back support belt of claim 5 wherein the edge inserts (13) are substantially triangular.

7. The back support belt of any one of the preceding claims wherein the strap (9) is supplemented by a secondary strap (12) of greater or lesser elasticity.

8. The back support belt of any one of the preceding claims wherein the strap (9) is in two parts (10,11) interconnected by mechanical fasteners.

## Patentansprüche

1. Rückenstützgürtel, der Folgendes umfasst:
ein orthopädisches Polster (1), das für eine Positionierung an der Lendenwirbelsäule gedacht und aus einem elastischen, im Wesentlichen aber nicht komprimierbaren, die Körperhaltung stützenden Material hergestellt ist und
eine konturierte, dem Körper zugewandte Oberfläche aufweist, die geeignet ist, sich an den normalen Hohlrücken im Beckenbereich des unteren Rückgrates anzupassen und/oder diesen aufrecht zu erhalten, und das eine vertikale Rille (2) in seiner Mitte aufweist, wobei die vertikale Rille geeignet ist, sich bei der Verwendung an das untere Rückgrat des Trägers anzupassen, und
einen Gurt (9), der sich von einander gegenüberliegenden Enden des orthopädischen Polsters (1) ausgehend erstreckt, um bei der Verwendung von dort um den Körper des Trägers herum gelegt zu werden,
**dadurch gekennzeichnet, dass** der Gürtel Folgendes umfasst:
vier Magnetstreifen (8), von denen zwei auf jeder Seite der vertikalen Rille (2) in der Mitte des orthopädischen Polsters angeordnet sind und parallel zur Rille (2) verlaufen, während die beiden anderen auf beiden Seiten der vertikalen Rille (2) angeordnet sind und senkrecht zu den betreffenden Magnetstreifen verlaufen, welche sich parallel zur Rille (2) erstrecken, wobei alle diese Magnetstreifen wechselnde Polaritäten besitzen, um so dabei zu helfen, dass die Blutgefäße des Trägers Felder kreuzen, die zwischen entgegen gesetzten magnetischen Polaritäten erzeugt sind, wodurch es dem Magnetfeld ermöglicht wird, geladene Teilchen im Blut anzuziehen und abzustoßen, um eine Bewegung und Wärme zu erzeugen.

2. Rückenstützgürtel nach Anspruch 1, der weiterhin Schlaufen und Taschen (18) für Werkzeuge und Geräte umfasst.

3. Rückenstützgürtel nach Anspruch 1 oder 2, bei dem der Gurt (9) im Wesentlichen unelastisch ist.

4. Rückenstützgürtel nach einem der vorhergehenden Ansprüche, bei dem der Gurt (9) weiterhin einen oder mehrere elastische Randeinsätze (13) umfasst.

5. Rückenstützgürtel nach Anspruch 4, bei dem die Randeinsätze (13) paarweise an seitlich einander gegenüberliegenden Stellen des Gurtes (9) vorgesehen sind.

6. Rückenstützgürtel nach Anspruch 5, bei dem die Randeinsätze (13) im Wesentlichen dreieckig sind.

7. Rückenstützgürtel nach einem der vorhergehenden Ansprüche, bei dem der Gurt (9) durch einen Sekundär-Gurt (12) für eine größere oder geringere Elastizität ergänzt ist.

8. Rückenstützgürtel nach einem der vorhergehenden Ansprüche, bei dem der Gurt (9) aus zwei Teilen (10, 11) besteht, die durch mechanische Befestigungsvorrichtungen miteinander verbunden sind.

## Revendications

1. Ceinture de maintien pour le dos, qui comprend :
un coussin orthopédique (1) destiné à être positionné contre la colonne lombaire, formé substantiellement en un matériau de maintien de la posture élastique mais sensiblement non compressif, ayant une surface faisant face au corps profilée, adaptée pour se conformer à et/ou maintenir une lordose lombaire normale de la colonne vertébrale inférieure, et ayant un sillon vertical (2) en son centre, le sillon vertical étant adapté pour accueillir la colonne vertébrale inférieure de l'utilisateur durant l'utilisation ; et
une sangle (9) s'étendant depuis des extrémités opposées du coussin orthopédique (1) pour l'extension depuis celui-ci autour du torse de l'utilisateur durant l'utilisation ;
**caractérisée en ce que** la ceinture comprend :
quatre bandes magnétiques (8), deux étant arrangées sur l'un ou l'autre côté du sillon vertical (2) au centre du coussin orthopédique et s'étendant parallèlement au sillon (2), les deux autres étant également arrangées sur l'un ou l'autre côté du sillon vertical (2) et étant perpendiculaires aux bandes magnétiques respectives s'étendant parallèlement au sillon (2), toutes les bandes magnétiques ayant des polarités alternées de manière à aider les vaisseaux sanguins de l'utilisateur à traverser les champs magnétiques créés entre les polarités magnétiques opposées, permettant ainsi au champ magnétique d'attirer et de repousser les particules chargées dans le sang pour créer des mouvements et de la chaleur.

2. Ceinture de maintien pour le dos selon la revendication 1, comprenant en outre des passants et des poches (18) pour les outils et les fournitures.

3. Ceinture de maintien pour le dos selon la revendication 1 ou 2, dans laquelle la sangle (9) est substantiellement non élastique.

4. Ceinture de maintien pour le dos selon l'une quelconque des revendications précédentes, dans laquelle la sangle (9) comprend en outre un ou plusieurs inserts marginaux élastiques (13).

5. Ceinture de maintien pour le dos selon la revendication 4, dans laquelle les inserts marginaux (13) sont fournis en paires à des positions latéralement opposées de la sangle (9).

6. Ceinture de maintien pour le dos selon la revendication 5, dans laquelle les inserts marginaux (13) sont substantiellement triangulaires.

7. Ceinture de maintien pour le dos selon l'une quelconque des revendications précédentes, dans laquelle la sangle (9) est complétée par une sangle secondaire (12) d'une élasticité supérieure ou inférieure.

8. Ceinture de maintien pour le dos selon l'une quelconque des revendications précédentes, dans laquelle la sangle (9) est en deux parties (10, 11) interconnectées par des éléments de fixation mécaniques.
